# EUROPEAN PATENT SPECIFICATION

(11) **EP 2 997 920 B1**
(45) Date of publication and mention of the grant of the patent: **10.10.2018**
(21) Application number: 15182816.7
(22) Date of filing: 27.08.2015
(51) Int. Cl.: A61B 18/00, A61B 18/12, A61B 18/14, A61B 5/00, A61B 90/30, A61B 18/16, A61B 17/32

(54) **CAVITATING ULTRASONIC SURGICAL ASPIRATOR WITH RF ELECTRODES**
KAVITIERENDES CHIRURGISCHES ULTRASCHALLABSAUGGERÄT MIT HF-ELEKTRODEN
ASPIRATEUR CHIRURGICAL ULTRASONIQUE DE CAVITATION AVEC ÉLECTRODES RF

(30) Priority: 28.08.2014 US 201414471381
(43) Date of publication of application: 23.03.2016
(73) Proprietor: Integra LifeSciences Corporation, Plainsboro, NJ 08536 (US)
(72) Inventor: LUDIN, Lev, Raynham, MA 02767-0350 (US); STULEN, Foster, Cincinnati, OH 45242 (US)
(74) Representative: Small, Gary James

(56) References cited:
- US-A1- 2002 007 200
- US-A1- 2003 163 131
- US-A1- 2005 143 730
- US-A1- 2005 187 512
- US-A1- 2010 191 173

## Description

### Field of the Invention

The present invention relates to a system including a surgical apparatus for ultrasonically fragmenting, aspirating, and electro surgically coagulating or ablating tissue at an operative site on a patient.

### Background

The application of ultrasonically vibrating surgical devices for fragmenting and removing unwanted tissue with precision and safety has led to the development of valuable surgical procedures, and the use of ultrasonic aspirators for the fragmentation and surgical removal of tissue from a body is well known. This device utilizes a hollow probe or tool that vibrates at frequencies at or above 20 kHz with tip amplitudes of up to 300 microns. When the vibrating tip is placed against viable or diseased tissue, the moving tip ablates the cells and causes them to fragment or otherwise emulsify in the irrigation fluid that is being added simultaneously. The emulsified fluid is then aspirated through the hollow probe and deposited in a canister for histological examination or disposal.

The advantage of excising tissue with this device is that the surgeon can remove the lesion in very thin layers defined by the number of cells. By slowly removing the tumor from the top down, he can clearly see when he is reaching healthy tissue allowing him to stop before substantial collateral damage occurs. This is extremely desirable in brain and spine surgery, where tissue does not regenerate. General surgeons have used the device as well for lesions of the liver and spleen, for the same reasons.

One side effect of any surgery is bleeding when the veins, arteries or capillaries are severed. Ultrasonic surgery is more sparing of blood vessels than steel (or stainless steel) scalpels because the collagen content of the vessels is more resistant to ultrasonic emulsion. However, the capillaries and small vessels will be compromised upon exposure to high amplitude ultrasonic tools. When these vessels are severed or punctured bleeding will of course occur. The surgeon will then be forced to pause the procedure, remove the ultrasonic tool from the site and generally reach for a cauterizing device of some type to close off the bleeder. Once coagulation has been achieved, then the surgeon can grab the ultrasonic tool, reposition it in the wound site and continue the removal of tissue. This situation repeats itself often in the course of the operation, lengthening the time of the procedure and coincidently the risk to the patient. It is therefore desired to find a way to cauterize tissue with the ultrasonic tool in place so the surgeon can stop bleeding with minimal downtime caused by switching tools and positions.

Several improvements to the basic design of the ultrasonic aspirator have been disclosed which allow some degree of cauterization subsequent to or simultaneously with ultrasonic ablation, for example US publication number 2003/163131 A1. Most center on the application of RF cautery currents to the tool or probe itself. However, these improvements have a limited cauterizing zone.

Accordingly, a need has arisen for an improved surgical procedure and apparatus which allows for greater flexibility in the size of the cauterizing zone defined by the RF electrodes.

### Summary

It is an object of the present invention to provide an ultrasonic and ablative medical treatment device having a cannula comprising an ultrasonic frequency vibrating tip with a flue disposed around the cannula. A first electrode can be disposed on an outer surface of the flue and an RF generator can provide a current to the first electrode. A current flow is formed between the electrode and the tip and the tip acts as a ground or current path to the electrode. The first electrode takes the shape of a complete circumferential ring.

In other examples, the first electrode can be removably disposed on the flue to allow the surgeon to "clip-on" the electrode at the position of their choice. The first electrode is movable along a length of the flue.

A further example includes a second electrode disposed on the outside of the flue and proximal to the first electrode. The RF generator can also provide a current to the second electrode, and a second current flow is formed between the first electrode and the second electrode. Also, the second electrode can be removable and movable along a length of the flue.

Another example of an ultrasonic and ablative medical treatment device also has a cannula with an ultrasonic frequency vibrating tip and a flue disposed around the cannula. A first electrode can be disposed on an outside of the flue along with a second electrode. The second electrode is also disposed on the outside of the flue proximal to the first electrode. An generator provides a current to the first and second electrodes, forming a current flow. Further, a second current flow can be formed between the first electrode and the tip.

Yet additional examples include at least one of the first and second electrodes are at least one of a complete circumferential ring, a partially circumferential ring, or a stave. At least one of the first and second electrodes is removably disposed on or movable along a length of the flue. The first and second electrodes can also be coaxial to each other.

Also disclosed is a method not claimed to be part of the invention for conducting a medical surgical procedure using an ultrasonic and ablative medical treatment device. The steps of the exemplary method include providing the ultrasonic medical treatment device having a cannula with a tip and a surrounding flue. A first electrode can be disposed on the flue proximate to the tip and at least the tip and the first electrode can be inserted into a patient. The method can vibrate the tip with an ultrasonic frequency to treat the patient and create a current flow between the first electrode and the tip, grounding the first electrode to the tip. An additional step can include cauterizing tissues in the patient using the current flow.

A further example of the method can have the steps of moving the first electrode relative to the tip, along the flue and altering the current flow based on the moved electrode's new position. Also, the disposing step can have the steps of disposing a second electrode on the flue proximal to the first electrode, creating a first current flow between the first and second electrodes, and creating a second current flow between the first electrode and the tip.

Also disclosed is a method not claimed to be part of the invention for conducting a medical surgical procedure using an ultrasonic and ablative medical treatment device, having the steps of providing the ultrasonic medical treatment device having a cannula with a tip and a surrounding flue and disposing a plurality of stave electrodes along an outside and a length of the flue. At least the tip and a portion of the stave electrodes can be inserted into a patient and the tip can be vibrated with an ultrasonic frequency to treat the patient. A current flow can be created between at least two of the plurality of stave electrodes and tissues can be cauterized in the patient using the current flow.

### Brief Description of the Drawings

This invention is described with particularity in the appended claims. The above and further aspects of this invention may be better understood by referring to the following description in conjunction with the accompanying drawings, in which like numerals indicate like structural elements and features in various figures. The drawings are not necessarily to scale, emphasis instead being placed upon illustrating the principles of the invention.

The drawing figures depict one or more implementations in accord with the present teachings, by way of example only, not by way of limitation. In the figures, like reference numerals refer to the same or similar elements.
Figure 1 is a partial cross-section of a CUSA device without the RF electrodes of the present invention;
Figure 2 is a cross-section of the cannula and flue of Figure 1;
Figure 3 is a cross-section of the cannula and flue with an example of a single RF electrode of the present invention;
Figure 4 is a cross-section of the cannula and flue with an example of a multiple RF electrode;
Figure 5 is a side view of the cannula and flue with an example of multiple, switchable RF electrodes;
Figure 6 is a side view of the cannula and flue with an example of stave RF electrodes;
Figure 7 is a cross-section of the cannula and flue with an example of an adjustable RF electrode;
Figure 8 is a flow chart of an example of a treatment method using the system of the present invention; and
Figure 9 is a flow chart of another example of a treatment method using the system of the present invention.

### Detailed Description

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

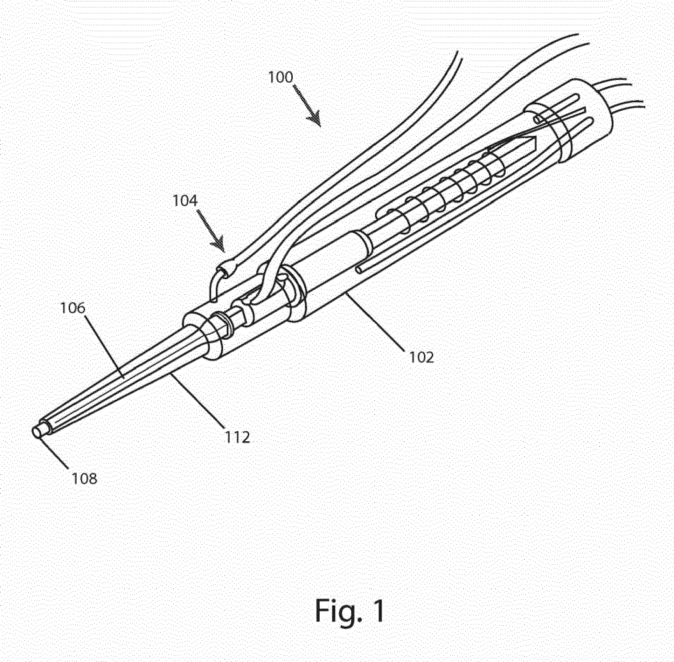

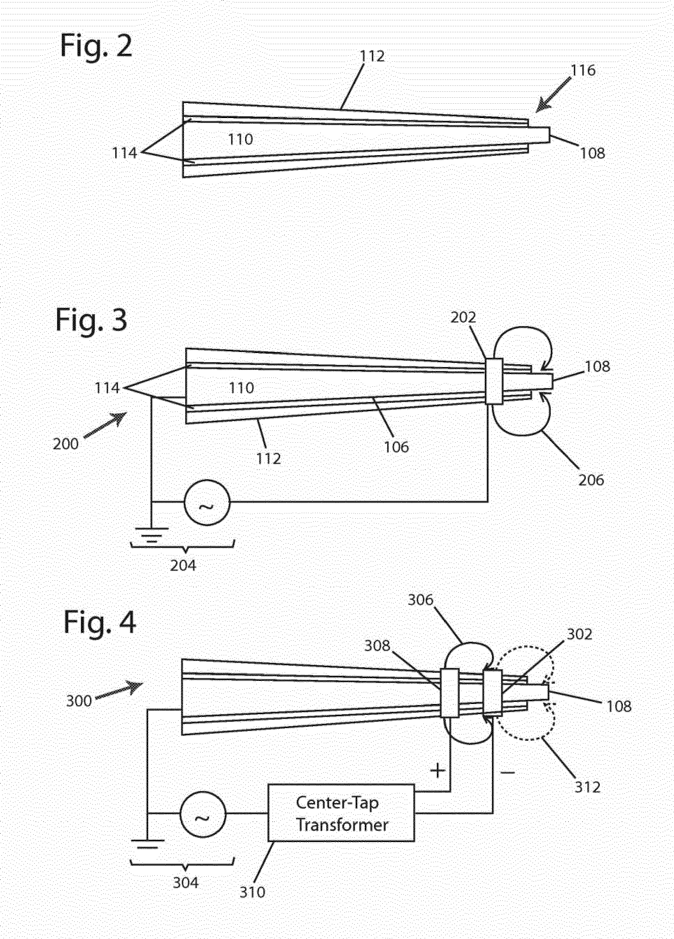

Figures 1 and 2 illustrate a prior art cavitating ultrasonic surgical aspirator ("CUSA") system 100 which includes a housing 102. The housing 102 can be part of a hand piece for manipulating the system 100 by the surgeon. At a distal end 104 of the housing is a cannula blade 106 with an ultrasonically-vibrating surgical tip 108. The tip 108 vibrates primarily longitudinally thereby fragmenting tissue it contacts. The level of vibration can be manually and continuously adjustable to vary the amplitude of the tip 108. The cannula blade 106 can be tubular and form a suction path 110 therein. The tip 108 can be replaceable.

Disposed over the cannula blade 106 is a protective flue 112. The flue's 112 inside diameter is slightly larger than the cannula blade 106 to form a gap between the two. The gap can act as an irrigation channel 114 when the system 100 is in operation. Further, in an example, the cannula 106 and the flue 112 can be coaxial.

In use, an ultrasonic generator (not illustrated) provides electrical energy at ultrasonic frequencies to create a vibrational stroke of the tip 108. Driving the tip 108 fragments and removes the tissue it comes in contact with. An irrigation system (not illustrated) controls a flow of sterile irrigating solution through the irrigation channel 114 and exits the fluid near the tip 108 where it enters the operating field and suspends fragmented particles of tissue. An aspiration system (not illustrated) applies suction through the suction path 110 to the hollow surgical tip 108 to aspirate the fluid through an end of the tip 108 and deposits the fluid and tissue in a disposable container (not illustrated).

Figure 3 illustrates the prior art CUSA system with an example of a radio frequency ("RF") electrode system 200 of the present invention. This example utilizes the flue 112 to locate a first RF electrode 202. The electrode 202 can be a complete circumferential ring disposed on an outer surface of the flue 112. The electrode 202 can be disposed by electroplating, gluing, or otherwise molding it into the flue 112. Further in this example, the flue 112 can be a polymer or any other type of electrical insulator, or have an insulating coating. Additionally, in other examples, the first electrode 202 can be disposed completely on the outer surface of the flue 112.

The first electrode 202 can be disposed very near a distal end 116 of the flue 112 and can be connected to an RF generator (or electrosurgical unit or "ESU") 204. The cannula 106 can now act as the return path or ground so a current flow 206 is set up between the electrode 202 and distal tip 108 of the cannula 106. The current flow 206 forms a coagulation zone just proximal to the tip 108 of the cannula 106. In a yet further example, the first electrode 202 does not extend past the tip 108 and/or the distal end 116 of the flue.

Figure 4 illustrates another example of an RF CUSA system 300 utilizing two electrodes, a first electrode 302 and a second electrode 308. As above, the electrodes 302, 308 can be fully circumferential rings. In this example, the RF generator 304 can drive the electrodes 302, 308 with opposite phases of RF input. There are various means known in the art to introduce opposite phases to the electrodes 302, 308 and those skilled in the art can use other phase inverter methods than those disclosed below. In one example, a simple way is to insert a center tap transformer 310 between the output of the ESU 304 and the electrodes 302, 308. Each end of an output coil of the transformer 310 is connected to one of the electrodes 302, 308. The center tap transformer 310 can then be connected to the cannula 106. This sets up a primary current 306 between the electrodes 302, 308 and forms a primary coagulation/ablation zone.

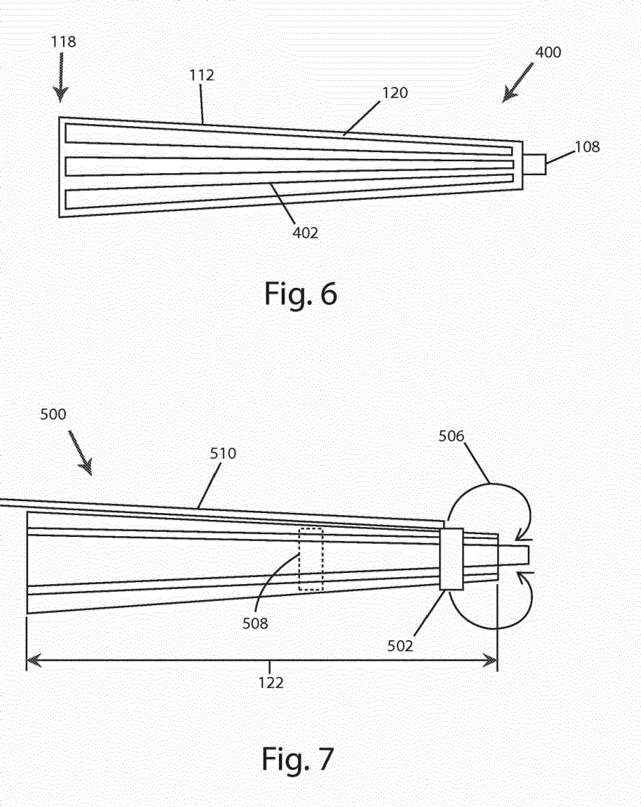

An additional example can include that the first and second electrodes 302, 308 are coaxial along a length 122 (see Figure 7) of the flue 112. In other words, the second electrode 308 is closer to the proximal end 118 of the flue 112 and the first electrode 302 is closer to the distal end 116 of the flue 112. Yet another way of describing it, the second electrode 308 is longitudinally "behind" or proximal to the first electrode 302 in relation to the tip 108.

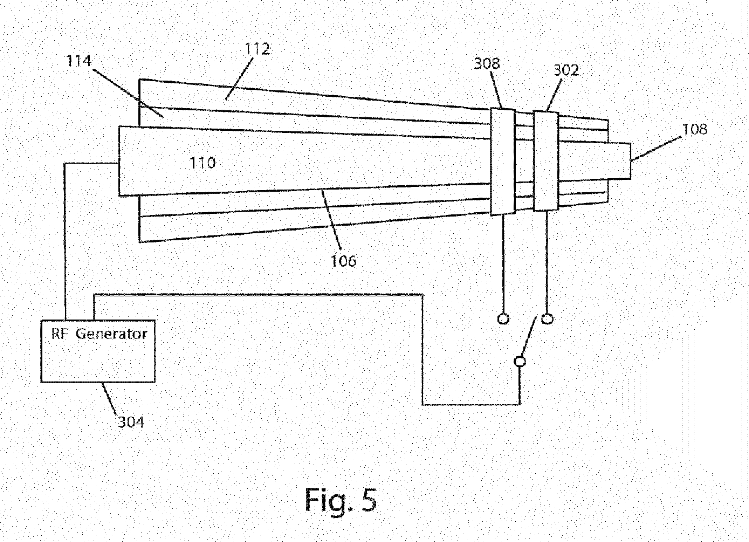

In a further example, some current may also flow to the cannula tip 108, forming a secondary current 312 and a secondary coagulation/ablation zone. The shape of the electrodes 302, 308, their spacing and distance to the tip 108 can be selected to enhance or reduce the amount of secondary current 312 going to the tip 108. With two electrodes 302, 308 an annulus of coagulation can be located anywhere along the flue 112. However, in a preferred example, an optimal position can be the distal end 116. In addition, Figure 5 illustrates first and second electrodes 302, 308 wherein the current can be switched between the two. Thus, a current flow can be formed between the first electrode 302 and the tip 108 or between the second electrode 308 and the tip 108. This allows the surgeon to control the size of the coagulation/ablation zone by just alternating between electrodes 302, 308. The region from the distal electrode and the tip is cauterized and the region between the distal and proximal electrodes can also be cauterized.

The energy can be delivered to tissue in contact with both poles of the electrical circuit. This allows the surgeon to concentrate the RF energy delivery on the bleeding surface and avoid surfaces or neighboring tissue where coagulation is not needed. In this case, instead of an annulus of coagulation forming, it can be just in the region of contact.

In the above examples, for symmetry, the electrodes 202, 302, 308 can be complete circumferential rings. Alternately, the electrodes 202, 302, 308 can be partially circumferential rings, with the distal and proximal partial electrodes 202, 302, 308 being aligned or offset. The electrodes 202, 302, 308 can also be split in a number of segments with multiple individual wires so that the individual segments can be turned on and off. This can be useful to deliver current only to the tissue immediately along a particular side of the flue 112.

Figure 6 illustrates another example of another system 400 with a different shape of the electrodes. The electrodes 402 can be longitudinal along the flue 112, rather than rings. The electrodes 402 can now be shaped similar to staves. A slope between the electrode staves 402 can be selected so that preferential current flow is located at the distal tip 108 or the proximal end of the flue 118. They can also be shaped to concentrate the current in a specific region, for example, at a center 120 of the flue 112. A further example can be that a number of stave electrodes 402 can be located around the flue 112 with each driven individually or in pairs (+/polarity). The pairs of stave electrodes 402 can be next to each other or on opposite sides of the flue 112 from each other.

Also, the number of electrodes 202, 302, 308, 402 can vary to form numerous patterns. One pattern can also be a spiral electrode winding up the flue 112.

Figure 7 illustrates an example of system 500 where the position of an electrode 502 can be adjustable along the length 122 of the flue 112. Adjustability allows the surgeon to adjust the coagulation zone by adjusting the current flow 506. The amount of the adjustment can be within the capability of the ESU 204, 304 to deliver sufficient power. Another adjustment example can be that the electrodes 502 are made of spring-type metal to elastically engage the flue 112 and be movable along its length. Note that in an example, the tip of the cannula 108 and/or the cannula 106 does not move in relation to the flue 112 and vice-a-versa.

There are at least two examples for the adjustable electrode 502. One can be that the electrode 502 is somewhat elastic so it can be slide up and down the flue 112 with a slider rod 510 or any other device capable of moving the electrode. The slider rod 510 can be connected to a control feature located on the handle 102. Another example is the use of two adjustable electrodes 502, 508. Here, either the first electrode 502 or the second electrode 508 can be moved along the flue 116 to change the distance between the first electrode 502 and the tip 108 or the distance between the first and second electrodes 502, 508. Alternately, both electrodes 502, 508 can be configured to move. The electrodes 502, 508 can move relative to each other, and the tip 108, along the length 122 of the flue 112.

The relative position of the first and second electrode determines the amount of current needed to accurately and rapidly coagulate the tissue. Therefore it is possible that the control feature for slider 510 in the handle could also be used to electronically set the nominal current driving the two electrodes. This allows the surgeon to set the desired power setting on an ESU and not need to make adjustments as the gap between the two electrodes change. Thus, in examples, the slider 510 can control different aspects of the current delivered. In one example, as the electrodes 502, 508 move relative to each other, the current can be changed as the gap between the electrodes 502, 508 change. This allows the surgeon to control the amount of current to be delivered. Alternately, or in addition to, the surgeon determines that she needs a larger coagulation zone, but want to maintain the current, the slider 510 can be used to again control the distance between the electrodes 502, 508 and at the same time control the amount of current to maintain the same current flow over the larger distance.

In one example, we understand that the coagulation zone can be both a function of the gap (i.e., the length between the electrodes 502, 508) and the lateral dimension of contact to the patient. The contact can be a full circumference of contact or a partial circumference of contact. In an example, the coagulation zone area can be the length between electrodes 502, 508 multiplied by half (1/2) of the circumference of the flue 112. This is based on the approximation that the surgeon is pressing the flue 112 against one side of the tissue.

A further example can be clip-on electrodes 502, 508 that can be placed by the surgeon. The clip-on electrode 502 geometry and their number and pattern can be unique for a given surgical step. Further, because the electrodes can be clipped-on, the electrodes can be manufactured, marketed and sold as an "after market" product. Thus, an existing CUSA only handset can be upgraded to include RF electrodes. As above, the number of electrode patterns can be huge, but another example is a pair of spiral wrapping electrodes. For the adjustable electrodes 502, 508, in an example, only the electrodes move and no other part of the system 500, including the flue 112 and the cannula 106.

In additional examples, when the electrodes 202, 302, 308, 402, 502, 508 are plated on the flue 112 they can have a thin conformal coating to protect them, in one example the plating can be gold. The wires leading to the electrodes 202, 302, 308, 402, 502, 508 in any of the examples, can be simply run to a single electrode. In a two or more electrode example, the wire to a distal electrode can run to a proximal electrode. A low profile example plates the wires connecting to the electrodes along the flue 112. These can be attached to a connector at the proximal end 118 of the flue 112 around the region where the irrigation tubing is connected. In the case above, a small gap in the more proximal electrode may be necessary for routing. Proper electrical clearance would need to be achieved between the distal electrode wire path and the size of the gap in the proximal electrode.

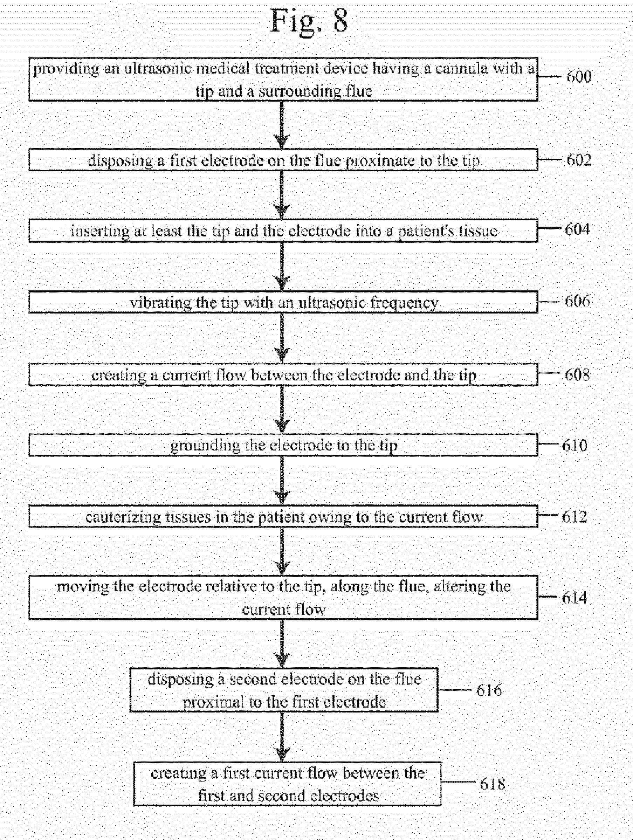

Figure 8 illustrates an example of a method for conducting a medical surgical procedure using an example of the RF CUSA as described above. The steps can include providing an ultrasonic medical treatment device having a cannula with a tip and a surrounding flue (step 600). Further steps include, disposing a first electrode on the flue proximate to the tip (step 602) and inserting at least the tip and the electrode into a patient (step 604). Next can be vibrating the tip with an ultrasonic frequency to treat the patient (step 606) and then creating a current flow between the electrode and the tip (step 608), which includes the step of grounding the electrode to the tip (step 610). Another step includes cauterizing tissues in the patient owing to the current flow (step 612). The power can be initiated by the surgeon when the need for additional hemostasis is identified. Typically this would be done with the foot switch supplied with most all ESUs

The method above can further have the step of moving the electrode relative to the tip, along the flue, altering the current flow (step 614). Additionally, the disposing step can include disposing a second electrode on the flue proximate to the first electrode (step 616) and the step of creating the current flow can include creating a first current flow between the first and second electrodes (step 618) and creating a second current flow between the first electrode and the tip (step 620).

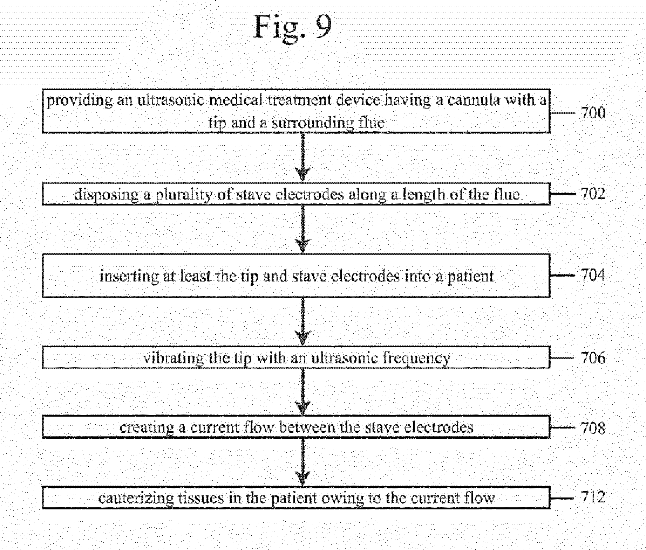

Figure 9 illustrates another example of a method for conducting a medical surgical procedure using an example of the RF CUSA as described above. The steps can include providing an ultrasonic medical treatment device having a cannula with a tip and a surrounding flue (step 700). Further steps include, disposing a plurality of stave electrodes along a length of the flue (step 702) and inserting at least the tip and a portion of the stave electrodes into a patient (step 704). Next can be vibrating the tip with an ultrasonic frequency to treat the patient (step 706) and then creating a current flow between at least two of the plurality of stave electrodes (step 708). Another step includes cauterizing tissues in the patient owing to the current flow (step 712).

The ring or stave electrodes incorporated or otherwise attached to the flue 112 can be used as conventional wire conductors. Adjunctive sensors, lights, indicators, etc. can be attached to the flue 112 and powered by the conductors. As an example, a white LED can be attached to flue 112 and the electrode can power the LED. In this case, DC power would be delivered to light the LED and AC RF would be delivered to achieve hemostasis.

While the foregoing has described what are considered to be the best mode and/or other examples, it is understood that various modifications may be made therein and that the subject matter disclosed herein may be implemented in various forms and examples, and that the teachings may be applied in numerous applications, only some of which have been described herein. It is intended by the following claims to claim any and all applications, modifications and variations that fall within the true scope of the present teachings.

## Claims

1. An ultrasonic and ablative medical treatment device (100), comprising:
a cannula (106) comprising an ultrasonic frequency vibrating tip (108);
a flue (112) disposed around the cannula (106);
a first electrode (202) disposed on an outer surface of the flue (112); and
an RF generator (204) providing a current to the first electrode (202);
wherein a current flow (206) is formed between the electrode (202) and the tip (108), and
wherein the tip (108) acts as a ground to the electrode (202), **characterised in that**:
the first electrode (202) is movable along a length of the flue (112) to adjust the length of a coagulation zone by adjusting the current flow (206), and
the first electrode (202) is a complete circumferential ring.

2. The medical device of claim 1, wherein the first electrode (202) is removably disposed on the flue (112).

## Patentansprüche

1. Ultraschall- und ablative medizinische Behandlungsvorrichtung (100), umfassend:
eine Kanüle (106), die eine Ultraschallfrequenz-Vibrationsspitze (108) umfasst,
einen Abzug (112), der um die Kanüle (106) herum angeordnet ist,
eine erste Elektrode (202), die an einer äußeren Fläche des Abzugs (112) angeordnet ist, und
einen HF-Generator (204), der die erste Elektrode (202) mit einem Strom versorgt,
wobei ein Stromfluss (206) zwischen der Elektrode (202) und der Spitze (108) gebildet ist und
wobei die Spitze (108) als Masse für die Elektrode (202) wirkt, **dadurch gekennzeichnet, dass**
die erste Elektrode (202) entlang einer Länge des Abzugs (112) beweglich ist, um durch Anpassen des Stromflusses (206) die Länge einer Koagulationszone anzupassen, und
die erste Elektrode (202) ein vollständiger Umfangsring ist.

2. Medizinische Vorrichtung nach Anspruch 1, wobei die erste Elektrode (202) entfernbar am Abzug (112) angeordnet ist.

## Revendications

1. Dispositif de traitement médical d'ablation ultrasonore (100) comprenant :
une canule (106) comprenant un embout vibrant à fréquence ultrasonore (108) ;
une gaine (112) disposée autour de la canule (106) ;
une première électrode (202) disposée sur une surface extérieure de la gaine (112) ; et
un générateur RF (204) fournissant un courant à la première électrode (202) ;
un flux de courant (206) étant formé entre l'électrode (202) et l'embout (108), et
l'embout (108) agissant comme une masse pour l'électrode (202), **caractérisé en ce que** :
la première électrode (202) est mobile le long d'une longueur de la gaine (112) afin d'ajuster la longueur d'une zone de coagulation en ajustant le flux de courant (206), et
la première électrode (202) est un anneau circonférentiel complet.

2. Dispositif médical selon la revendication 1, dans lequel la première électrode (202) est disposée de manière amovible sur la gaine (112).
